# EUROPÄISCHE PATENTANMELDUNG

(11) **EP 3 954 418 A1**
(43) Veröffentlichungstag der Anmeldung: **16.02.2022**
(21) Anmeldenummer: 20190395.2
(22) Anmeldetag: 11.08.2020
(51) Int. Cl.: A61M 16/00, A61M 16/20, A61M 16/08

(54) **ATEMWEGSTHERAPIEGERÄT**

(71) Anmelder: CEGLA Medizintechnik GmbH & Co. KG, 56410 Montabaur (DE)
(72) Erfinder: Cegla, Ulrich Hartmann, 56410 Montabaur (DE); Ebinger, Andrea, 56410 Montabaur (DE)
(74) Vertreter: Engelhardt, Volker

(57) **Zusammenfassung**

Bei einem Atemwegstherapiegerät (1) zur Behandlung der Muskulatur von Atemwegen, Lungenfunktionen und/oder Schleimablagerungen im Lungen-, Nasen- und/oder Rachenraum eines Patienten (2),
bestehend aus:
- einem Hohlkörper (3), in dem mindestens ein Durchgangskanal (4, 3, 5) vorgesehen ist,
- einem in den Hohlkörper (3) eingesetztes Mund- oder Nasenstück (6), das mit dem Patienten (2) während der Behandlungsdauer des Atemwegstherapiegerätes (1) verbunden ist,
- und aus mindestens einem im Inneren des Hohlkörpers (3) angeordneter Schwingkörper (11, 12), der durch die Ein- und/oder Ausatmung des Patienten (2) in eine oszillierende Vibration versetzt ist,
sollen durch das Atemwegstherapiegerät (1) eine oszillierende, resistive und/oder Threshold typische Luftdruckschwankungen erzeugbar sein, bei dem die individuellen Widerstände bei der Ein- und Ausatmung individuell patientengerecht einstellbar ist und eine Umrüstung oder Umstellung des Atemwegstherapiegeräts (1) nicht erforderlich ist. Dies ist dadurch erreicht, dass der Schwingkörper (11) als Halteplatte (13) mit einer Luftaustrittsöffnung (14) ausgestaltet ist, dass an der Halteplatte (13) eine frei schwingende Zunge (15) befestigt ist, durch die die Luftaustrittsöffnung (7, 8) durch die Luftaustrittsöffnung (14) der Halteplatte (13) in Abhängigkeit von den im Inneren des Hohlkörpers (3) vorherrschenden Luftströmungen (7, 8) verschlossen oder bereichsweise freigegeben ist und/oder dass der Schwingkörper (12) als plattenförmiges Ventil (16, 17) ausgestaltet ist, dessen eine Seite bereichsweise an dem Hohlkörper (3) befestigt und dessen dazu gegenliegende Seite frei schwingend angeordnet ist.

## Beschreibung

Die Erfindung bezieht sich auf ein Atemwegstherapiegerät zur Behandlung der Muskulatur von Atemwegen, Lungenfunktionen und/oder zur Schleimlösung im Lungen-, Nasen- und/oder Rachenraum eines Patienten nach dem Oberbegriff des Patentanspruchs 1.

Ein solches Atemwegstherapiegerät ist beispielsweise in EP 3 251 718 B1 beschrieben. In einem Rohrstück ist dort ein biegeelastischer Körper in Form eines Schlauches an einem Mundstück befestigt. Das Rohrstück ist gekrümmt, sodass der Schlauch eine Krümmung und folglich eine Querschnittsverringerung im Krümmungsbereich aufweist. Sobald ein Patient durch das Mundstück Atemluft einsaugt oder auspresst, wird die Umgebungsluft bzw. die Atemluft durch den Schlauch gezogen bzw. gedrückt, sodass dieser in eine Schwingung oder Vibration gelangt. Durch diese Vibration entstehen oszillierende Luftdruckschwankungen im Mund-, Nasenraum und/oder der Lunge des Patienten, wodurch sowohl die Muskulatur der Atemwege als auch die Schleimlösung in diesen Bereichen entsteht.

Durch die EP 3 620 195 A1 ist eine Beatmungs-Vorrichtung bekannt geworden, die dazu dient, einen künstlich beatmeten Patienten zu versorgen und gleichzeitig die Verschleimung der Bronchien zu lösen und gleichzeitig die Verschleimung der Bronchien zu lösen sowie die Atemmuskulatur zu trainieren. Diese Vorrichtung besteht aus einem Atemwegstherapiegerät, beispielsweise der eingangs zitierten Gattung.

Solche Atemwegstherapiegeräte haben sich in der Praxis seit Jahrzehnten bewährt und werden zur Behandlung von Patienten erfolgreich verwendet. Gleichwohl hat sich nachteiliger Weise herausgestellt, dass der verwendete Schlauch zum einen eine sehr geringe Lebensdauer aufweist und zum anderen bereits nach wenigen Atemzügen verstopfen kann. Darüber hinaus werden im Inneren des Schlauches Aerosolen, Schleim oder sonstige Bakterien bzw. Viren abgelagert, die nach jeder Behandlung zu entfernen sind. Somit ist der Schlauch nach wenigen Behandlungsvorgängen aufwendig aus dem Rohrstück und dem Mundstück zu entnehmen und beispielsweise in einer Mikrowelle oder in einem sonstigen zur Sterilisation geeigneten medizinischen Gerät zu verbringen, um eine solche Keimlösung zu erreichen.

Wie jede Muskulatur kann auch die Atemmuskulatur trainiert werden. Beim Muskeltraining unterscheiden die Mediziner zwischen einem Stärketraining und einem Ausdauertraining. Die bisherigen Geräte zum Training der Atemmuskulatur sind entweder resistive (Widerstand erzeugende Geräte) oder sogenannte Thresholdgeräte. Bei Thresholdgeräten öffnet sich bei Überwindung eines gewissen Druckes ein Federventil und gibt eine Öffnung frei.

Werden resistive Atemmuskeltrainer verwendet, dann wird in der Regel der Atmung eine Stenose vorgeschaltet.

Zum Training der Muskulatur muss ein gewisser Widerstand überwunden werden. Wenn ein vergleichbares Training durchgeführt werden soll, muss dieser Widerstand bei jedem Training verständlicherweise identisch sein.
Alle bisher auf den Markt gekommenen Geräte sind weitgehend für die Ein- oder Ausatmung bestimmt und verfügen ausschließlich nur über statische Druckerzeugung (PIP =POSITIVE INSPIRATORY PRESSURE).

Ein Atemtrainingsgerät, das inspiratorisch oszillierend, resistive (OPIP) und thresholdgetrieben die Bronchien erweitert und darin festsitzenden Schleim löst oder verflüssigt, gibt es im Zusammenhang mit dem Atemmuskeltraining bisher somit nicht.

Der Erfindung liegt daher die Aufgabe zugrunde, ein Atemwegstherapiegerät der eingangs genannten Gattung derart weiterzubilden, dass eine oszillierende, resistive und/oder threshold typische Luftdruckschwingungen entstehen lässt, bei dem die individuellen Widerstände bei der Ein- und Ausatmung individuell Patienten gerecht, einstellbar ist und eine Umrüstung oder Umstellung des Atemwegstherapiegerätes nicht erforderlich ist.

Je nach Länge, Schwere, Steifigkeit und Lage der Zunge sowie der Ausgestaltung des Trainingsgeräteraums kann die Trainingslast zusätzlich individuell angepasst werden.

Diese Aufgabe ist durch die Merkmale des kennzeichnenden Teils von Patentanspruch 1 gelöst.

Weitere vorteilhafte Weiterbildungen der Erfindung ergeben sich aus den Unteransprüchen.

Dadurch, dass der Schwingkörper als Halteplatte mit einer Luftaustrittsöffnung ausgestaltet ist, und dass an der Halteplatte eine frei schwingende Zunge befestigt ist. Bei einem Unter- bzw. Überdruck an der Halteplatte kommt es zu einer Auslenkung der Stimmzunge, die ähnlich einem Ventil die Luft nach außen strömen lässt. Der Schwingkörper kann dabei eine auf-bzw. durchschlagende Zunge besitzen, so dass die Ventilfunktion nur in eine bzw. in beide Richtungen funktioniert. Durch diesen Mechanismus werden oszillierende Druckschwankungen verschiedenster Form erzeugt, die zur Bronchialerweiterung, stabilisierend und Schleim lösend wirksam sind, durch die die Luftaustrittsöffnung der Halteplatte in Abhängigkeit von den im Inneren des Hohlkörpers vorherrschenden Luftströmungen verschlossen oder bereichsweise freigegeben ist und/oder dass der Schwingkörper als plattenförmiges Ventil ausgestaltet ist, dessen eine Seite bereichsweise an dem Hohlkörper befestigt und dessen dazu gegenliegende Seite frei schwingend angeordnet ist, entsteht ein Atemwegstherapiegerät für individuelle medizinische Atemmuskulatur-Trainingszwecke, bei dem beim Einsaugen und/oder Ausatmen der Atemluft sowohl eine oszillierende, eine resistive oder threshold getriebene Bronchienerweiterung zur Verfügung gestellt ist.

Der Erfindungsgegenstand verwendet verschiedene Zungen zur Produktion der in- und expiratorischen Oszillationen. Eine solche Zunge hat einen definierten Fluss bzw. Druck, bei dem diese zu schwingen beginnt und damit Oszillationen erzeugt werden. Unterschiedliche Töne werden dann hörbar. Diese Drucke und Flüsse werden bei der Ein- bzw. Ausatmung erzeugt und können so die Ein- bzw. Ausatmung genau unterscheiden und entsprechend aufzeichnen. Durch Vorschaltung unterschiedlich definierte Stenosen, kann dieser Druck bzw. Fluss, bei dem die Zungen zu schwingen beginnen, eingestellt werden, so dass bei Erreichen es definierten Druckes ein Ton erzeugt wird. Bei Nutzung des Gerätes als Muskeltrainer während der Einatmung wird das Training bei einer einstellbaren "Mindestgegenkraft" durchführbar.

Werden die Zungen senkrecht (vertikal) im Gerät verbaut, ist der aufzuwendende Druck geringer als wenn die Zungen unter einem Winkel kleiner oder größer 90 Grad verbaut werden. Um eine Umrechnung des Druckes zu vermeiden, soll die Zunge senkrecht verbaut werden.

Da der Atemwegswiderstand definiert ist als Druckänderung dividiert durch Flussänderung, muss eine für den Patienten erkennbare Kennzeichnung der Erreichung des gewünschten Druckes bestehen, da sonst durch unterschiedliche Flüsse bei identischer Stenose unterschiedliche Drucke vorhanden sind und damit eine unterschiedliche Trainingslast entsteht.

Der Schwingungsbeginn einer Stimmzunge ist durch deren Steifigkeit und Masse bedingt. Gleiche Stimmzungen haben bei definierter Strömung den identischen Schwingungsbeginn. Die unterschiedlichen Durchmesser von Stenosen haben bei gleicher Strömung unterschiedliche Drucke zur Folge. Die Kombination einer Stimmzunge mit Nachschaltung einer Stenose definierten Durchmessers erzeugt damit bei Schwingungsbeginn der Zunge ein reproduzierbares definiertes Druckintegral. Dies kann akustisch wahrgenommen und damit aufgezeichnet werden.

Gegendieses Druckintegral muss die Atemmuskulatur arbeiten. Darüber wird sie definiert und reproduzierbar trainiert.

Von der Steifigkeit und der Länge der Zunge sowie der Verbauungswinkel hängt die Kraft ab, die notwendig ist, um die Zunge zum Schwingen zu bringen und einen Ton zu erzeugen. Wenn diese Kraft bekannt ist, kann bei vorgegebener Stenose der Widerstand, der erreicht wird, exakt bestimmt werden. Da die Steifigkeit der Zunge sich nicht ändert, ist der Druck, bei dem diese zu schwingen beginnt, stets identisch. Somit lässt sich für alle Stenosen deren "kritischer Druck" und damit der überwundene Widerstand genau bestimmen.

Neben der Änderung des Einbauwinkels kann auch die Auswahl der unterschiedlichen Stenosen, zu einem unterschiedlichen Druck führen, bei dem die Zunge zu schwingen beginnt oder die Durchgangsöffnung der Stenose offen ist.

Durch die entstehenden Druckschwankungen infolge der Schwingungen der Zunge werden die Bronchien erweitert und der Bronchialschleim, da er thixotrop ist, von den Wänden abgeschert sowie verflüssigt.

Bei Verwendung einer aufschlagenden Zunge als "Druckerzeuger und -anzeiger" ohne Stenose entsteht durch die Schwingung ein Druckbild, das dem dynamischen PEP (zero-to-peak; peak-to-zero) gleichkommt, das heißt der Druck steigt von 0 auf ein Maximum, um dann sofort wieder auf 0 zu fallen. Neben der Länge der Stimmzunge ist auch deren Form für ein bestimmtes Druckbild verantwortlich. Je länger und schwerer bzw. steifer die Zunge ist, umso höher ist auch der Initialdruck. Die Zunge muss genügend Platz in der Verbauung haben, damit der Lösabstand eingehalten werden kann, um die Zunge zum Aufschwingen zu bekommen.

Je länger die Zunge ist, umso mehr Abstand benötigt diese. Für die dynamische Druckform werden daher lange und schwere Stimmzungen verarbeitet. Die entstehende Druckform wird zur Bewegung des Bronchialschleimes, zum Abscheren und Verflüssigen, benötigt.

Wird statt der aufschlagenden Zunge eine durchschlagende Zunge benutzt, die ebenfalls bei Tonerzeugung das Erreichen eines bestimmten Druckes anzeigt, entspricht das Druckschwankungsbild hier einem kombinierten PEP. Die Druckschwankung setzt sich auf einen dauerpositiven Druck auf.

Neben der Platte mit Zunge kann auch ein Schwingkörper als Ventil mit dieser kombiniert sein. Die Stenose bleibt vorgeschaltet, um diesen kombinierten PEP zu erreichen. Diese Druckform ist zur Erweiterung der Bronchien und zur Verbesserung der kollateralen Ventilation (u.a. Eröffnung der kollateralen Atemwege) in der Lunge notwendig.

Durch die Verwendung von zwei unterschiedlichen Schwingkörpern, kann dann zudem rein akustisch zwischen In- und Expiration unterschieden werden. Diese Unterschiede können mithilfe einer App bzgl. Häufigkeit, Länge und OPIP bzw. OPEP-Anwendung (unterschiedliche Oberschwingungen) aufgenommen, ausgewertet und dokumentiert werden.

Eine zusätzliche variable Stenose kann zwischen Mundstück und der Zungen führenden Röhren geschaltet werden.

Mittels der erfindungsgemäßen Anordnung von unterschiedlich ausgestalteten Schwingkörpern in einem Hohlkörper, der beispielsweise auch mit einem Inhalationsgerät oder auch in ein Schlauchsystem eines Beatmungsgerätes eingesetzt ist, kann folglich jeder Zustand eines Patienten berücksichtigt sein. Bei künstlich beatmeten Patienten kann das erfindungsgemäß beschriebene Atemwegstherapiegerät in den Beatmungskreislauf ein- und ausgeschaltet werden. So kann der Bronchialschleim gelöst und verflüssigt werden sowie die Atemmuskulatur leicht trainiert werden.

In der Zeichnung sind sieben Ausführungsvarianten eines Atemwegstherapiegeräts dargestellt, die nachfolgend näher erläutert sind. Im Einzelnen zeigt:
- Figur 1: ein erstes Ausführungsbeispiel, eines Atemwegstherapiegeräts, das aus einem Hohlkörper und einem an diesem befestigten Mundstück besteht und durch das ein Patient Atemluft aus der Umgebung einsaugt, wobei das freie Ende des Hohlkörpers mit einer Stenose, in der drei Durchgangsöffnungen mit unterschiedlichen Öffnungsquerschnitten eingearbeitet sind und verkleinert ist, wobei ein erster Schwingkörper in einem Durchgangskanal des Hohlkörpers angeordnet ist, in Seitenansicht,
- Figur 2a: eine Vergrößerung des ersten Schwingkörpers in Form einer Halteplatte, in die eine rechteckförmige Luftaustrittsöffnung eingearbeitet ist, die von einer Zunge abgedeckt ist, im angehobenen Zustand gemäß Figur 1,
- Figur 2b: die Halteplatte gemäß Figur 2b mit abgesenkter Zunge, durch die die Austrittsöffnung verschlossen ist,
- Figur 2c: eine Verlängerung der Luftaustrittsöffnung mit einer die Luftaustrittsöffnung in beide Richtungen durchschlagende Zunge,
- Figur 3a: ein zweites Ausführungsbeispiel eines Atemwegstherapiegeräts, das sowohl zur Einatmung als auch zur Ausatmung verwendet werden kann, mit zwei ineinander mündenden Hohlkörpern, deren jeweilige freie Stirnseiten mit einer der Stenosen verschlossen ist und mit drei im Inneren der Hohlkörper angeordneten Schwingkörpern in Form einer Halteplatte mit Zunge bzw. eines Ventils, während des Einatmungsvorganges,
- Figur 3b: das Atemwegstherapiegerät gemäß Figur 3a, während des Ausatmungsvorgangs,
- Figur 4: ein drittes Ausführungsbeispiel eines Atemwegstherapiegeräts, in dem die Schwingkörper schräg zur jeweiligen Symmetrieachse der Hohlkörper angeordnet sind oder orthogonal zu diesen verlaufen,
- Figur 5: das Atemwegstherapiegerät gemäß Figur 1, an dessen Schwingkörper Sensoren und Mikrofone angeschlossen sind, die mit einem externen Gerät kommunizieren,
- Figur 6a: ein viertes Ausführungsbeispiel eines Atemwegstherapiegeräts, in dessen Hohlkörper eine Trennwand vorgesehen ist, durch die der Hohlkörper in zwei Durchgangskanäle unterteilt ist, durch die die Atemluft beim Einatmen strömt, in denen jeweils mindestens eine der beiden Schwingkörper eingebaut ist,
- Figur 6b: das Atemwegstherapiegerät gemäß Figur 6a, durch die die ausgeatmete Atemluft durch den zweiten Durchgangskanal in die Umgebung zurückgedrückt ist,
- Figur 7: ein fünftes Ausführungsbeispiel eines Atemwegstherapiegerätes in Weiterbildung der Ausführungsversion gemäß den Figuren 6a und 6b mit zwei in dem jeweiligen Durchgangskanal vorgesehenen Schwimmkörpern,
- Figur 8: ein sechstes Ausführungsbeispiel eines Atemwegstherapiegeräts mit zwei in den jeweiligen Durchgangskanälen des Atemwegstherapiegeräts gemäß Figur 6a angeordneten Schwimmkörpern in orthogonaler bzw. schräger Anordnung, und
- Figur 9: ein siebtes Ausführungsbeispiel eines Atemwegstherapiegerätes gemäß Figur 1, das in einem Schlauch eingesetzt ist, wobei der Schlauch Bestandteil einer Beatmungs-Vorrichtung ist, durch die ein künstlich zu beatmender Patient mit Sauerstoff versorgt ist.

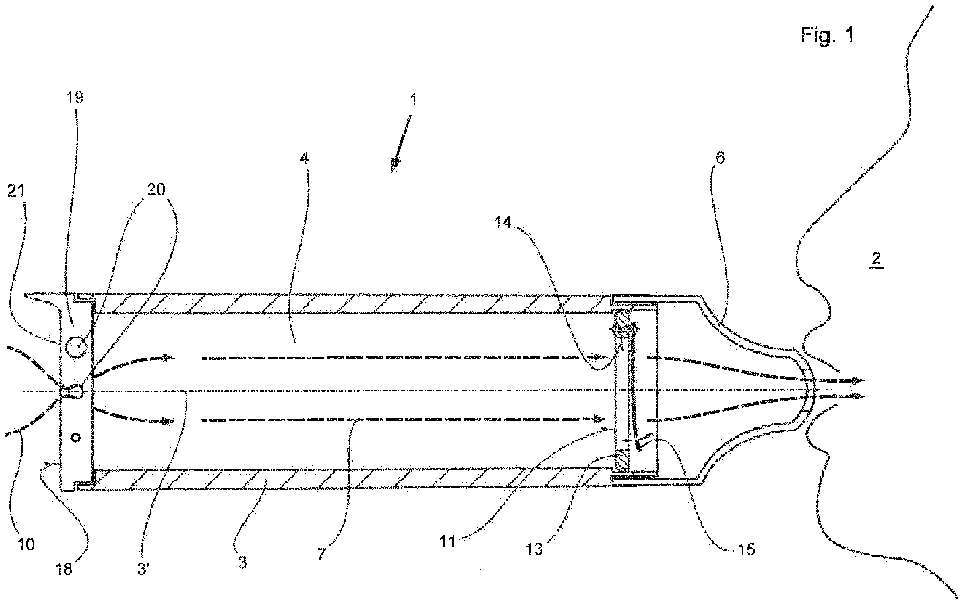

In Figur 1 ist ein Atemwegstherapiegerät 1 abgebildet, durch das ein Patient 2 seine Atemmuskulatur zur Schleimlösung in den Bronchien und/oder seine Lungenfunktion verbessern kann. Das Atemwegstherapiegerät 1 besteht aus einem Hohlkörper 3, der beispielsweise als Rohrstück ausgestaltet ist. An einem der freien Enden des Hohlkörpers 3 ist ein Mundstück 6 vorgesehen, das mit dem Patienten 2 verbunden werden kann, sodass der Patient 2 durch seine Atemmuskulatur durch das Mundstück 6 Umgebungsluft einsaugen kann.

Das freie dem Mundstück 6 gegenüberliegende Stirnseite des Hohlkörpers 3 ist mit einer Stenose 18 verschlossen. In die Mantelfläche 19 der Stenose 18 sind drei Durchgangsöffnungen 20 mit unterschiedlich groß bemessenen Durchmessern eingearbeitet. Sobald der Patient 2 durch das Mundstück 3 Umgebungsluft 7 einsaugt, strömt diese durch die Durchgangsöffnungen 20 in das Innere des Hohlkörpers 3, der folglich als Durchgangskanal 4 für die eingesaugte Umgebungsluft 7 dient. Die derart in das Innere des Hohlkörpers 3 eingesaugte Umgebungsluft ist mit der Bezugsziffer 7 gekennzeichnet. Aufgrund der Anzahl und der vorhandene Durchmesser der Durchgangsöffnungen 20 benötigt der Patient 2 bereits eine gesteigerte Atemkraft, um die Umgebungsluft 7 überhaupt in den Durchgangskanal 4 einzusaugen.

Da die Atemluft möglichst vibrierende oder oszillierende Drücke aufbauen soll, ist unmittelbar vor dem Mundstück 6 im Durchgangskanal 4 ein erster Schwingkörper 11 angeordnet.

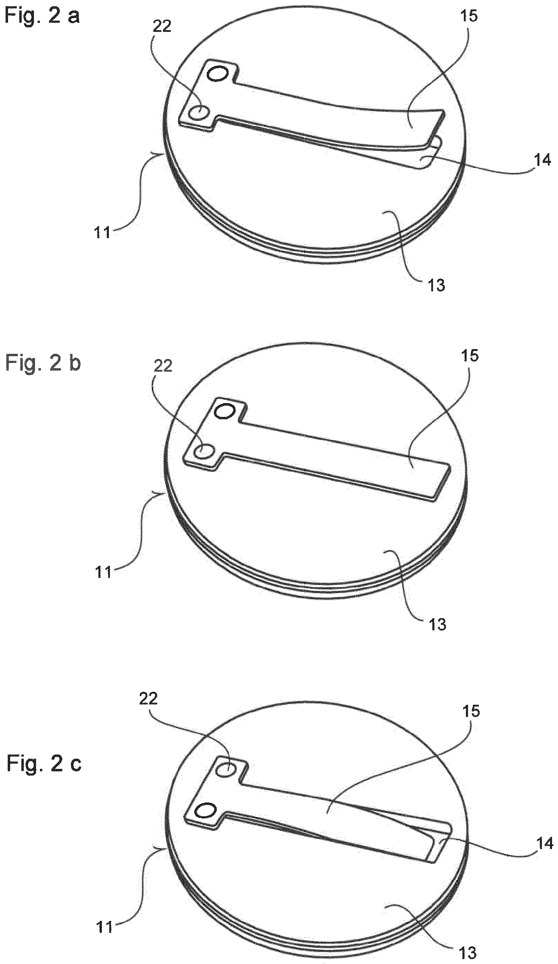

Gemäß Figur 2a umfasst der erste Schwingkörper 11 eine Halteplatte 13, in die eine rechteckförmige Luftaustrittsöffnung 14 eingearbeitet ist. Die Luftaustrittsöffnung 14 ist von einer Zunge 15 bereichsweise im unbetätigten Zustand verschlossen. Ein freies Ende der Zunge 15 ist dabei mittels zweier Schrauben 22 an der Halteplatte 13 befestigt. Die Außenkontur der Halteplatte 13 ist dabei an die in Kontur des Durchgangskanals 4 des Hohlkörpers 3 angepasst, sodass die eingesaugte Atemluft 7 ausschließlich durch die Luftaustrittsöffnung 14 in Richtung des Mundstückes 6 strömen kann, denn die Mantelfläche der Halteplatte 13 liegt luftdicht an der Innenwand des Durchgangskanals 4 an. Die Zunge 15 schwingt demnach in Richtung des Mundstücks 6, sobald der Patient 2 Atemluft 7 durch das Mundstück 6 saugt. Somit ist die Zunge 15 an der freien Stirnseite, die den Schrauben 22 gegenüberliegt, durch den vom Patienten 2 aufgebrachten Einatmungsdruck angehoben und die Austrittsöffnung 14 ist bereichsweise freigegeben.

Sobald der Einatmungsvorgang durch den Patienten 2 beendet ist, federt die Zunge 15 gemäß Figur 2b auf die Oberseite der Halteplatte 13 zurück und die Luftaustrittsöffnung 14 wird folglich vollständig verschlossen, sodass diese luftdicht abgedichtet ist. Die von der Zunge 15 erzeugten vibrierenden oder oszillierenden Luftdruckschwankungen hängen folglich von der Biegesteifigkeit der Zunge 15 und der Masse der Zunge 15 sowie den Abmessungen des Durchmessers der Stenose 18 bzw. der Luftaustrittsöffnung 14 ab.

In Figur 2c ist gezeigt, dass die Austrittsöffnung 14 verlängert werden kann, sodass die Länge der Zunge 15 kleiner bemessen ist als die Länge der Luftaustrittsöffnung 14. Dies bedeutet, dass zwischen dem freien Ende der Zunge 15 und der Innenseite der Luftaustrittsöffnung 14 ein Luftspalt entsteht und die Zunge 15 in beide Richtungen durch die Luftaustrittsöffnung 14 schwingen oder tauchen kann. Folglich schwingt die Zunge 15 durch die Luftaustrittsöffnung 14 und somit kann das Atemwegstherapiegerät 1 gemäß Figur 1 bei einer derartigen Ausgestaltung des Schwingkörpers 11 sowohl zum Einatmen als auch zum Ausatmen verwendet werden.

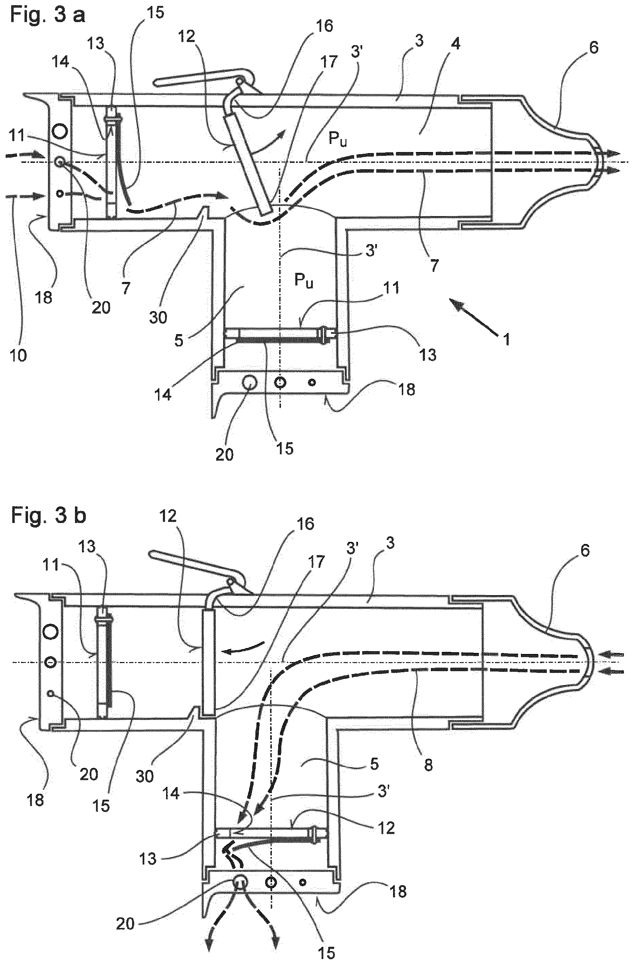

In den Figuren 3a und 3b ist eine andersartige Ausgestaltung des Atemwegstherapiegeräts 1 zu entnehmen, das nunmehr zwei miteinander verbundene Hohlkörper 3 aufweist, deren jeweilige mit der Bezugsziffer 3' gekennzeichneten Symmetrieachsen senkrecht zueinander verlaufen. Die jeweiligen freien Stirnseiten der beiden Hohlkörper 3 sind mittels der Stenose 18 verschlossen. In dem ersten Durchgangskanal 4 sind zwei Schwingkörper 11 und 12 angeordnet.

Der erste Schwingkörper 11 umfasst die Halteplatte 13 mit der Luftaustrittsöffnung 14, die von der Zunge 15 gemäß Figur 2a und 2b verschlossen bzw. teilweise freigegeben ist. Darüber hinaus ist in dem Durchgangskanal 4 ein zweiter Schwingkörper 12 angeordnet, dessen erste Stirnseite mit dem Hohlkörper 3 verschwenkbar mittels eines Drehgelenkes 16 verbunden ist. Das gegenüberliegende freie Ende des Schwingkörpers 12 kann sich von einem an der Innenseite des Hohlkörpers 3 angeformten Anschlag 30 abheben und ist mit der Bezugsziffer 17 gekennzeichnet, wenn der Patient 2 Umgebungsluft durch die Stenose 18 in den Durchgangskanal 4 einsaugt. Der zweite Schwingkörper 11 wirkt als eine Art Ventil.

Die Zunge 15 des ersten Schwingkörpers 11 hebt sich dabei von der Luftaustrittsöffnung 14 ab. In dem Durchlasskanal 4 entsteht ein Unterdruck, der mit Pu gekennzeichnet ist. Da die beiden Durchgangskanäle 4 und 5 miteinander verbunden sind, entsteht im zweiten Durchgangskanal 5 der Unterdruck Pu, wodurch die Zunge 15 des ersten Schwingkörpers 11 auf die Halteplatte 13 gedrückt ist, um die Luftaustrittsöffnung 14 zu verschließen. Die Zunge 15 ist nämlich auf der dem ersten Durchgangskanal 4 abgewandten Seite der Halteplatte 13 angeordnet, sodass der Unterdruck Pu die Zunge 15 auf die Halteplatte 13 zieht.

Beim Ausatmen gemäß Figur 3b werden die beiden Schwingkörper 11 und 12 im ersten Durchgangskanal 4 verschlossen, denn das Ventil 16 wird gegen den Anschlag 30 beim Ausatmen durch die Atemluft 8 gedrückt; ebenso verschließt sich die Luftaustrittsöffnung 14, da die Zunge 15 auf die Halteplatte 13 aufgrund der vorherrschenden Vorspannkraft der Zunge 15 gedrückt ist und somit auf diese aufliegt. Die ausgeatmete Atemluft 8 strömt demnach durch das Mundstück 6 in den ersten Durchgangskanal 4 und anschließend in den zweiten Durchgangskanal 5, sodass die Atemluft 8 auf die Zunge 15 des im Durchgangskanal 5 angeordneten Schwingkörper 11 auftrifft und dabei die Zunge 15 bereichsweise von der Luftaustrittsöffnung 14 abhebt bzw. aufdrückt. Die Atemluft 8 strömt anschließend durch die in der freien Stirnseite des zweiten Hohlkörpers 3 angeordneten Durchgangsöffnung 20 der Stenose 18 in die Umgebung.

Folglich kann das Atemwegstherapiegerät 1 gemäß den Figuren 3a und 3b sowohl zum Einatmen als auch zum Ausatmen verwendet werden. Durch die zwei unterschiedlich wählbaren Stenosen 18 für Ein- und Ausatmung lässt sich die Trainingslast für diese beiden Atemmanöver individuell und getrennt einstellen.

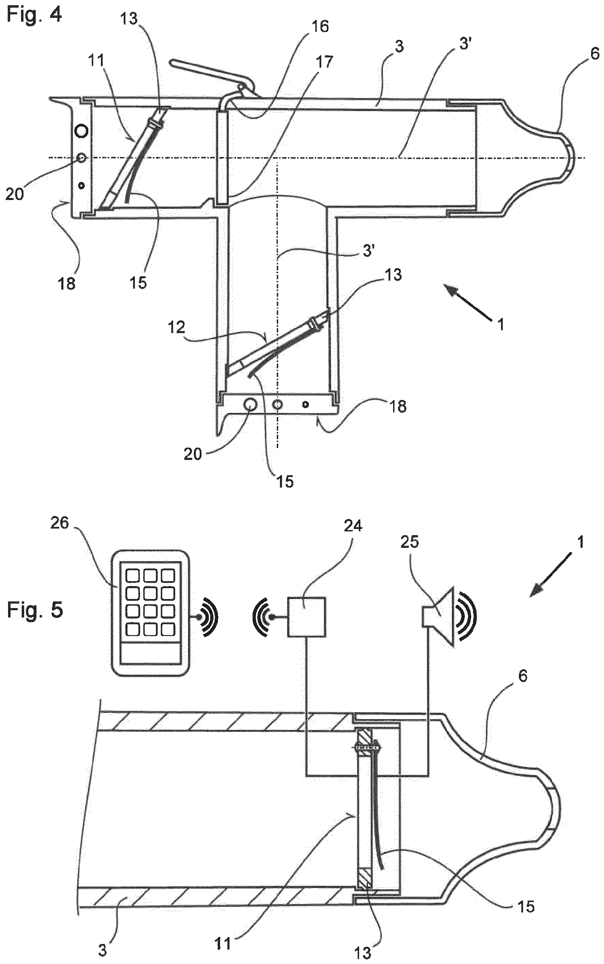

In Figur 4 ist eine andersartige Anordnung der Schwingkörper 11 in den beiden Hohlkörpern 3 dargestellt. Gemäß den Figuren 3a und 3b verlaufen die Schwimmkörper 11 senkrecht zu der Symmetrieachse 3' des jeweiligen Hohlkörpers 3 und in Figur 4 ist zu entnehmen, dass die Schwingkörper 11 in einem vorgegebenen Winkel, also geneigt zu der jeweiligen Symmetrieachse 3', angeordnet sind.

Gemäß Figur 5 soll der Schwingkörper 11 sowohl als akustische als auch als elektronische Übertragungseinrichtung genutzt werden. Daher sind beispielsweise an der Zunge 15 zugeordnete Lautsprecher 25 oder elektrische Sensoren 24 vorgesehen. Die Lautsprecher 25 übertragen dabei einen Ton, sodass der Patient 2 während der Benutzung des Atemtrainers (Inspiration) bzw. Atemwegstherapiegeräts (Expiration) 1 akustisch wahrnehmen kann, ob seine Atemmuskulatur die Zunge 15 ausreichend in Schwingung versetzt und wie lange dieser den oszillierenden Widerstand, also die vorgegebene Trainingslast halten kann.

Die Sensoren 24 kommunizieren beispielsweise über eine WLAN-Verbindung mit einem externen Gerät 26. Dabei kann das Gerät 26 die Benutzungsdauer, die verwendeten Frequenzen der Zunge 15 und die Anzahl der Atemübungen des Patienten 2 aufzeichnen, sodass diese beispielsweise von einem Arzt nach einer bestimmten Zeitspanne ausgelesen und ausgewertet werden können.

Werden dann noch zwei verschiedene Zungen 15 eingebaut, kann direkt zwischen dem Training der Einatemmuskulatur inklusive der entsprechenden Trainingslast bzw. bei der Ausatmung die Dauer inklusive der resistiven und thresholdgetriebenen Therapiehöhe unterschieden und aufgezeichnet werden. Für diese Aufzeichnungen kann auch über eine APP ein herkömmliches Handy genutzt werden, so dass Lautsprecher und Mikrofon überflüssig werden könnten.

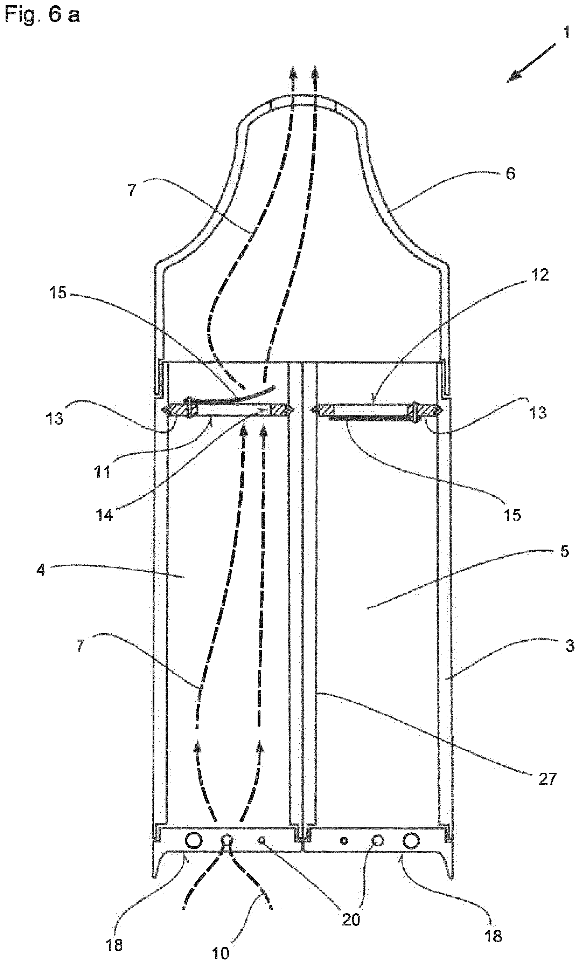

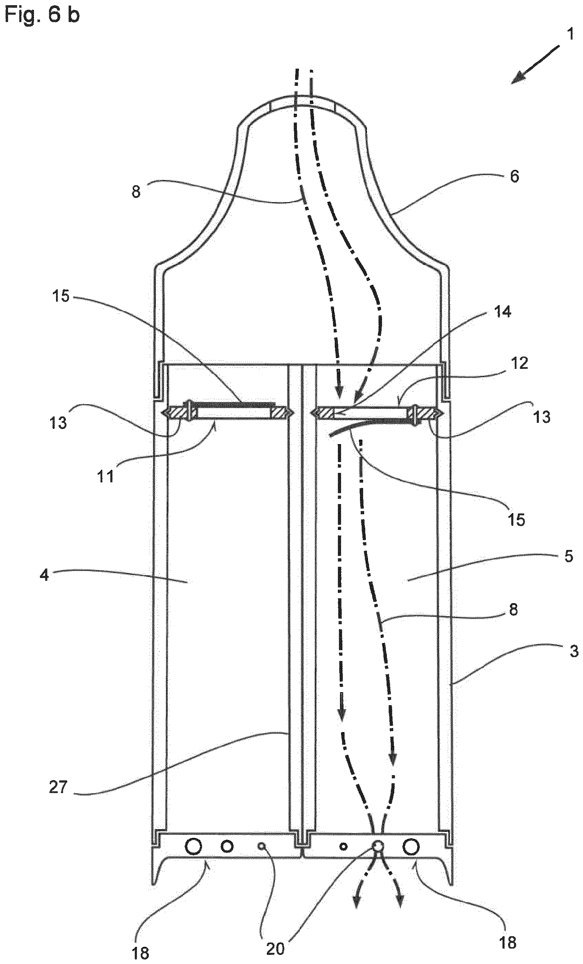

In den Figuren 6a und 6b umfasst der Atemtrainer bzw. das Atemwegstherapiegerät 1 einen Hohlkörper 3, in dem eine Trennwand 27 zur Bildung von zwei luftdicht voneinander getrennten Durchgangskanälen 4 und 5 gebildet ist. Die jeweiligen freien Stirnseiten des Hohlkörpers 3 sind mittels der Stenose 18 teilweise verschlossen. In jedem der Durchgangskanäle 4 und 5 ist einer der Schwingkörper 11 angeordnet.

Gemäß Figur 6a ist der im Durchgangskanal 4 vorgesehene Schwimmkörper 11 geöffnet, sobald der Patient Atemluft 7 durch den Durchgangskanal 4 saugt, da die Zunge 15 in Richtung des Mundstückes 6 durch die eingesaugte Atemluft 7 aufgedrückt ist. Die Zunge 15 ist auf der dem Mundstück 6 zugewandten Oberseite der Halteplatte 13 angeordnet.

Gemäß Figur 6b öffnet sich die Zunge 15 im Durchgangskanal 5, sobald der Patient 2 ausatmet, da die Atemluft 8 zunächst die Zunge 15 des Schwingkörpers 11 im Durchgangskanal 4 auf die Halteplatte 13 drückt und folglich der Durchgangskanal 4 verschlossen ist und die Atemluft 8 die Zunge 15 des im Durchgangskanal 5 angeordneten Schwingkörper 11 aufdrückt. Die Zunge 15 ist auf der dem Mundstück 6 abgewandten Oberseite der Halteplatte 13 angeordnet.

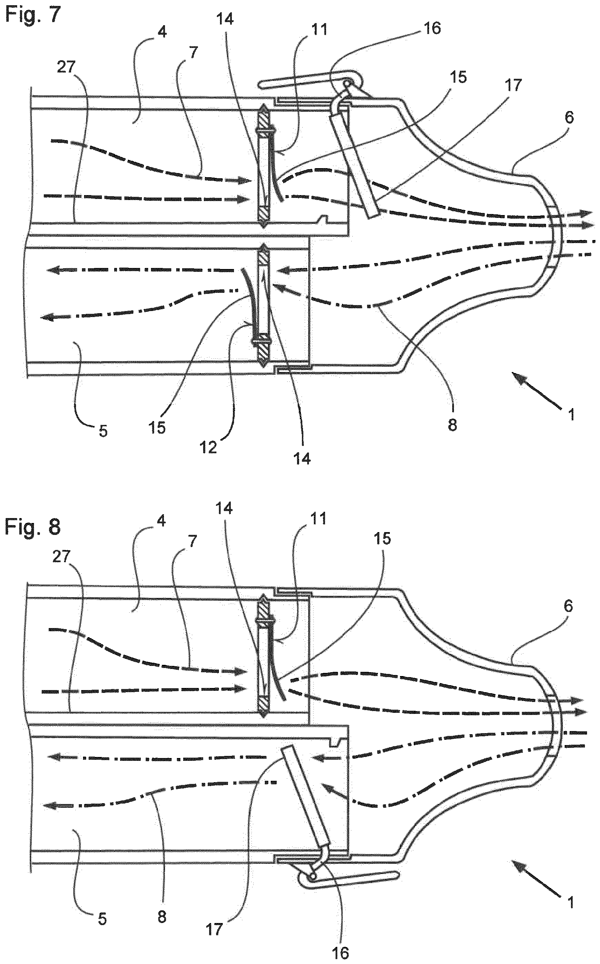

In Figur 7 ist eine Kombination aus erstem Schwingkörper 11 mit durchschlagender Zunge 15 und zweitem Schwingkörper 12 abgebildet. Der Durchgangskanal 4 ist dabei sowohl mittels des Schwingkörpers 11 und des Schwingkörpers 12 beim Einatmen geöffnet und beim Ausatmen sind diese durch die Atemluft 8 verschlossen, sodass die Atemluft 8 den Schwingkörper 11 im Durchgangskanal 5 aufdrückt.

Es ist mölgich, eine Kombination aus Inhalationsgerät / Vernebler 36 mit zweitem Schwingkörper 12 zu benutzen. Der Durchgangskanal 4 ist dabei mittels des Schwingkörpers 12 beim Einatmen geöffnet und beim Ausatmen ist dieser durch die Atemluft 8 verschlossen, so dass die Atemluft 8 den Schwingkörper 11 im Durchgangskanal 5 aufdrückt.

In Figur 8 ist gezeigt, dass der Durchgangskanal 4 mittels eines Schwingkörpers 11 und der Durchgangskanal 5 mittels eines Schwingkörpers 12 verschlossen ist. Wahlweise können diese Kombinationen entsprechend ausgetauscht sein und die Ausrichtung der Schwingkörpers 11 und 12 bezogen auf die Symmetrieachse 3' des Hohlkörpers 3 können senkrecht oder geneigt zu dieser verlaufen.

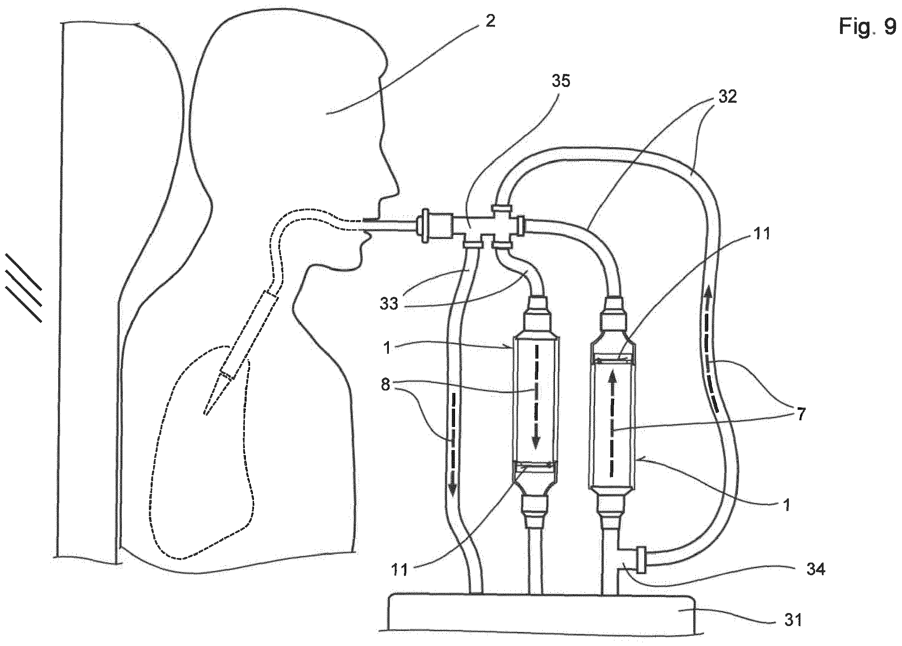

Aus Figur 9a ist ersichtlich, dass beispielsweise die Ausführungsvariante nach Figur 1 in einem System zur Beatmung des Patienten 2 einsetzbar ist, um die Atemmuskulatur des beatmenden Patienten 2 zu trainieren und den Schleimabtransport zu erleichtern. Dabei kann das erfindungsgemäße Atemwegstherapiegerät 1 sowohl in einem Einatemschenkel 32 eingebaut sein.

Durch einen Luftverteiler 34 (2-Wegehahn) kann die Beatmungsmaschine 31 entweder den Patienten 2 über den Einatemkanal 32 mit dem Atemtherapiegerät 1 (Druckschwankungen und positiver Druck oder bei Umschaltung direkt das Tracheostoma für die Beatmung ansteuern. In dem Atemtherapiegerät muss dann die Zunge 15 ein bisschen geöffnet gezeichnet werden. Der Luftstrom 8 geht nur durch das Atemtherapiegerät 1. Die Expiration läuft rein über den Ausatemschenkel.

Es ist üblich, die Schaltung des Atemtherapiegerätes im Ausatemschenkel bei einem beatmeten Patienten an dessen Gesundheitszustand anzupassen. Hier findet sich am Tracheostoma ein Ventil 34 (2-Wegehahn), der die Ausatemluft entweder über den Ausatemschenkel inkl. Atemtherapiegerät 1 (8a) oder über den Ausatemschenkel 8b des Beatmungssystems leitet. Hier sollte die Zunge ein wenig nach unter gezeichnet werden.

Somit ist das beschriebene Atemwegstherapiegerät 1 auf vielfältige Weise einsetzbar. Die erläuterten Anordnungen von den Schwingkörpern 11, 12 sowie die Stenose 18 in dem Hohlkörper 9 können beliebig positioniert sein, solange die Schwingkörper 11, 12 den jeweiligen Durchlasskanal 4, 5 freigeben bzw. verschließen.

## Patentansprüche

1. Atemwegstherapiegerät (1) zur Behandlung der Muskulatur von Atemwegen, Lungenfunktionen und/oder Schleimablagerungen im Lungen-, Nasen- und/oder Rachenraum eines Patienten (2),
bestehend aus:
- einem Hohlkörper (3), in dem mindestens ein Durchgangskanal (4, 3, 5) vorgesehen ist,
- einem in den Hohlkörper (3) eingesetztes Mund- oder Nasenstück (6), das mit dem Patienten (2) während der Behandlungsdauer des Atemwegstherapiegerätes (1) verbunden ist,
- und aus mindestens einem im Inneren des Hohlkörpers (3) angeordneter Schwingkörper (11, 12), der durch die Ein- und/oder Ausatmung des Patienten (2) in eine oszillierende Vibration versetzt ist,
**dadurch gekennzeichnet,**
**dass** der Schwingkörper (11) als Halteplatte (13) mit einer Luftaustrittsöffnung (14) ausgestaltet ist, dass an der Halteplatte (13) eine frei schwingende Zunge (15) befestigt ist, durch die die Luftaustrittsöffnung (7, 8) durch die Luftaustrittsöffnung (14) der Halteplatte (13) in Abhängigkeit von den im Inneren des Hohlkörpers (3) vorherrschenden Luftströmungen (7, 8) verschlossen oder bereichsweise freigegeben ist und/oder dass der Schwingkörper (12) als plattenförmiges Ventil (16, 17) ausgestaltet ist, dessen eine Seite bereichsweise an dem Hohlkörper (3) befestigt und dessen dazu gegenliegende Seite frei schwingend angeordnet ist.

2. Atemwegstherapiegerät nach Anspruch 1,
**dadurch gekennzeichnet,**
**dass** die dem Mundstück (6) gegenüberliegende Stirnseite (9) des Hohlkörpers (3) offen ist und dass in diese Stirnseite (9) Stenosen (18) eingesetzt sind, in deren Mantelfläche (19) mindestens eine Durchgangsöffnung (20) vorgesehen ist, durch die Umgebungsluft (10) in das Innere des Hohlkörpers (3) strömt oder vice versa austritt.

3. Atemwegstherapiegerät nach Anspruch 2,
**dadurch gekennzeichnet,**
**dass** die Stenose (18) eine Wand (21) aufweist, durch die der Zugang in den Hohlkörper (3) in Richtung dessen Symmetrieachse (3') verschlossen ist, und dass die Außenkontur der Stenose (18) an der Innenkontur des Hohlkörpers (3) angepasst ist.

4. Atemwegstherapiegerät nach einem der Ansprüche 2 oder 3,
**dadurch gekennzeichnet,**
**dass** die Durchgangsöffnungen (20) unterschiedlich groß bemessen sind.

5. Atemwegstherapiegerät nach einem der Ansprüche 2 oder 4,
**dadurch gekennzeichnet,**
**dass** die Durchgangsöffnungen (20) mittels eines Stopfens (23) verschließbar sind.

6. Atemwegstherapiegerät nach einem der vorgenannten Ansprüche,
**dadurch gekennzeichnet,**
**dass** die Zunge (15) einseitig auf der Oberfläche der Halteplatte (13) aufliegt bzw. abhebt oder dass die Zunge (15) durch die Luftaustrittsöffnung (14) oszillierend Luft einströmen und/oder ausströmen lässt.

7. Atemwegstherapiegerät nach einem der vorgenannten Ansprüche,
**dadurch gekennzeichnet,**
**dass** der erste und zweite Schwingkörper (11 ,12) in einer Atemrichtung und/oder in entgegengesetzter Atemrichtung angeordnet sind.

8. Atemwegstherapiegerät nach einem der vorgenannten Ansprüche,
**dadurch gekennzeichnet,**
**dass** der erste und/oder zweite Schwingkörper (11, 12) orthogonal oder in einem vorgegebenen Winkel bezogen auf die Steigungsrichtung der Atemluft (7, 8) angeordnet sind.

9. Atemwegstherapiegerät nach einem der vorgenannten Ansprüche,
**dadurch gekennzeichnet,**
**dass** an mindestens einem der Schwingkörper (11, 12) ein Sensor (24) und/oder ein Mikrofon (25) angeschlossen ist, wodurch akustische oder elektrische Signale an ein externes Gerät (26) übertragen sind.

10. Atemwegstherapiegerät nach einem der vorgenannten Ansprüche,
**dadurch gekennzeichnet,**
**dass** das Atemwegstherapiegerät (1) in einem Beatmungs- und/oder Ausatmungs-Schlauch (32, 33) einer Beatmungs-Vorrichtung (31) eingebaut ist.

11. Atemwegstherapiegerät nach einem der vorgenannten Ansprüche,
**dadurch gekennzeichnet,**
**dass** die Luftströmungen (7, 8) der Beatmungs-Vorrichtung (31) über einen Luftverteiler (34) und einem Mehrwege-Ventil (35) derart steuerbar sind, dass wahlweise der Patient (2) die ein- und/oder ausgeatmete Atemluft (7, 8) durch das in den Schläuchen (32,33) eingesetzte Atemwegstherapiegerät (1) ein- und/oder auspresst oder ausschließlich mit der Beatmungs-Vorrichtung (31) gekoppelt ist.
